# EUROPÄISCHE PATENTSCHRIFT

(11) **EP 2 404 561 B1**
(45) Veröffentlichungstag und Bekanntmachung des Hinweises auf die Patenterteilung: **01.04.2015**
(21) Anmeldenummer: 11008119.7
(22) Anmeldetag: 22.08.2005
(51) Int. Cl.: A61B 17/221

(54) **Vorrichtung zur Entfernung von Thromben**
Device for removing blood clots
Dispositif de suppression de thrombi

(30) Priorität: 23.08.2004 DE 102004040868
(43) Veröffentlichungstag der Anmeldung: 11.01.2012
(62) Teilanmeldung aus: 05777177.6
(73) Patentinhaber: Phenox GmbH, 44801 Bochum (DE)
(72) Erfinder: Hannes, Ralf, 44137 Dortmund (DE); Henkes, Hans Dr., 70192 Stuttgart (DE); Miloslavski, Elina Dr., 70192 Stuttgart (DE); Monstadt, Hermann Dr., 44797 Bochum (DE)
(74) Vertreter: Thiel, Christian

(56) Entgegenhaltungen:
- WO-A1-02/055146
- DE-A1- 3 921 071
- US-A- 5 899 850
- US-A1- 2002 072 764
- US-A1- 2004 199 202

## Beschreibung

Die Erfindung betrifft eine Vorrichtung zur Entfernung von Fremdkörpern und Thromben aus Körperhohlräumen und Blutgefäßen mit einem Führungsdraht, der ein distales Element aufweist, und mit einem Korbelement. Das distale Element ist unlösbar mit dem Führungsdraht verbunden.

Thromboembolische Erkrankungen wie Herzinfarkt, Lungenembolie, periphere Thrombose, Organembolien, etc. werden typischerweise durch einen Thromboembolus (im folgenden kurz Thrombus), also einen viskoelastischen Blutklumpen aus Blutplättchen, Fibrinogen, Gerinnungsfaktoren etc., ausgelöst, der sich in einem Blutgefäß festgesetzt hat und dieses ganz oder teilweise verschließt. Der Verschluß von Organarterien führt dabei zu einer Unterbrechung der Versorgung des abhängigen Gewebes mit Sauerstoff und Nährstoffen. Der Störung des Funktionstoffwechsels mit Funktionsverlust folgt innerhalb kurzer Zeit das Erliegen des Strukturstoffwechsels mit dem Untergang des betroffenen Gewebes (Infarkt). Die häufigsten hiervon beim Menschen betroffenen Organe sind das Herz und das Gehirn. Solche Veränderungen betreffen aber auch die Extremitätenarterien und die Lungenarterien. Venöse Thrombosen und thrombembolische Verschlüsse kommen gehäuft in den Bein- und Beckenvenen vor. Das Krankheitsbild des thrombotischen Verschlusses eines intrakraniellen Sinus kann durch die Störung der venösen Drainage des Hirngewebes zu schweren Hirnblutungen führen.

Angesichts der Schwere der durch Thromboembolien ausgelösten Krankheitsbilder und der Häufigkeit dieser Erkrankungen sind verschiedene Techniken zur Auflösung oder Entfernung von Thromben bekannt.

So ist es bekannt, solche Patienten mit thrombolytischen Mitteln wie Streptokinase oder Urokinase oder mit Antikoagulantien zu behandeln, was zur Thrombolyse oder zur Eindämmung des Thrombenwachstums führen soll. Da diese Behandlungsmethoden meist zeitintensiv sind, werden sie oftmals zusammen mit Eingriffen kombiniert, die der mechanischen Zerkleinerung oder Entfernung des Thrombus bzw. Embolus dienen.

Neben offenen chirurgischen Eingriffen kommen im Stand der Technik zunehmend transluminale bzw. endovaskuläre, Katheter geführte interventionelle Therapieformen zum Einsatz, da diese weniger invasiv sind. So ist es bekannt, den Thrombus mittels Unterdruck erzeugenden Saug-Kathetern oder mechanisch mit Kathetern, welche mit Fangkörben, Wendeln, Haken oder dergleichen versehen sind, aus dem Körper des Patienten zu entfernen, siehe US 6 245 089 B1, US 5 171 233 A1, Thomas E. Mayer et al., Stroke 2002 (9), 2232.

Der Nachteil der bekannten transluminalen Vorrichtungen besteht darin, daß auch diese den Thrombus häufig nicht vollständig entfernen können, und die Gefahr besteht, daß der Thrombus oder Fragmente davon sich freisetzen und im Blutstrom zu kleinlumigeren Gefäßen weiterziehen, welche schwerer zu erreichen und zu behandeln sind. Des weiteren eignen sich die im Stande der Technik bekannten Vorrichtungen aufgrund ihrer Dimensionen und/oder geringen Flexibilität nur ungenügend zur Entfernung von Thromben aus besonders kleinlumigen oder stark gewundenen Gefäßen, wie denen des Gehirns.

So ist aus der US 2002/0049452 eine Vorrichtung mit einem Katheter zur Entfernung von Thromben bekannt, an dessen distalem Ende Fangarme aus einem Formgedächtnismaterial angebracht sind, die im komprimierten Zustand am Katheter anliegen und sich in der expandierten Konfiguration radial vom Katheter nach außen erstrecken. Nach der durch die Körpertemperatur ausgelösten Einnahme der expandierten Konfiguration sollen sich die Fangarme im Thrombus verhaken und diesen bei Rückführung des Katheters in einen weiteren Katheter aus dem Blutgefäß mit sich ziehen. Der Nachteil dieser Vorrichtung besteht darin, daß sie entweder zur Kühlung der Fangarme unter die Umwandlungstemperatur, bis diese in den Blutstrom gelangen, in einem Sekundärkatheter, der diese Kühlung ermöglicht, an dem Thrombus vorbei manövriert werden muß; oder der mit den Fangarmen versehene Katheter muß in seinem Inneren eine Heizvorrichtung tragen, die die Erhitzung auf die Umwandlungstemperatur nach dem Erreichen des Thrombus ermöglicht. Diese konstruktiven Erfordernisse sind zum einen sehr aufwendig und somit auch störanfällig und machen allein aufgrund ihres physikalischen Umfanges die Behandlung besonders kleinlumiger Gefäße unmöglich.

US 2002/0072764 A1 offenbart eine Vorrichtung gemäß dem Oberbegriff des Anspruchs 1.

Angesichts der mit dem Stand der Technik verbundenen Nachteile besteht die Aufgabe der Erfindung somit in der Bereitstellung einer Vorrichtung zur Entfernung von Fremdkörpern und Thromben aus Körperhohlräumen und Blutgefäßen, welche das operative Risiko bei der Entfernung von Thromben vermindert und die Behandlung besonders kleinlumiger Gefäße erlaubt.

Diese Aufgabe wird erfindungsgemäß durch eine Vorrichtung gemäß Anspruch 1 gelöst, wobei das distale Element mit einer im wesentlichen orthogonalen Struktur ausgestattet ist. Vorzugsweise besteht die orthogonale Struktur aus einer Vielzahl von Fasern oder Borsten, die einzeln oder in Bündeln angeordnet sein können.

Die Erfindung beruht auf Ergebnissen der Erfinder, die belegen, daß die Verwendung einer derartig einfach konzipierten und somit besonders kleinvolumig haltbaren Vorrichtung geeignet ist, Thromben aus insbesondere auch sehr kleinlumigen Gefäßen zu bergen. Der als Einführhilfe ausgestaltete Führungsdraht ermöglicht die gute Manövrierbarkeit in auch kleinlumige und gewundene Gefäßabschnitte und hält den Gesamtdurchmesser der Vorrichtung, insbesondere aber den nicht variablen Durchmesser der Vorrichtung klein (der durch die Fasern bedingte Durchmesser ist insofern variabel, als diese biegsam sind, und kann daher auch verengte Gefäßpassagen passieren). Die Fasern/Borsten sind geeignet, einen Thrombus festzuhalten und zu stabilisieren, insbesondere dann, wenn sie aus thrombogenen Materialien bestehen oder damit ausgerüstet sind.

Es versteht sich, daß Führungsdraht und distales Element unlösbar miteinander verbunden sind.

Die Vorrichtung wird dabei mit Hilfe eines kleinlumigen Mikrokatheters an den Einsatzort gebracht. Es ist gleichermaßen möglich, die Vorrichtung im Mikrokatheter 1) zuerst distal des Thrombus zu manövrieren und dann zurückzuziehen, 2) im Bereich des Thrombus aus dem Mikrokatheter freizusetzen, 3) proximal des Thrombus aus dem Mikrokatheter herauszuschieben und dann anterograd den Thrombus zu penetrieren. Beim Vorschieben der Vorrichtung legen sich die biegsamen Fasern aufgrund des mechanischen Widerstandes in proximale Richtung an das distale Element an. Beim Zurückziehen dagegen stellen sie sich auf, verhaken sich im Thrombus und unterstützen so dessen Rückführung in einen größeren als den ursprünglich verwendeten Mikrokatheter. Entsprechend der aktuellen Technik wird man einen Führungskatheter verwenden, mit dem das betreffende Haupgefäß sondiert wird. Durch diesen Führungskatheter wird man einen Mikrokatheter koaxial einführen, der zur Einbringung der besagten Vorrichtung in die Zielregion dient. Den mit der besagten Vorrichtung fixierten Thrombus wird man vorzugsweise bis in den Führungskatheter zurückziehen und dann mit diesem aus dem Körper entfernen.

Es versteht sich, daß die Fasern/Borsten über eine hinreichende Steifigkeit verfügen, um ihren Zweck zu erfüllen, gleichzeitig aber auch hinreichend flexibel oder biegsam sind, um durch einen Katheter geführt zu werden und Gefäßwände nicht zu verletzen.

Die Fasern können aus einem Naturstoff, Polymermaterial, Metall, keramischem Material, Glas oder aus Kombinationen davon bestehen.

Gemäß einer bevorzugten Ausführungsform der Vorrichtung bestehen die Fasern aus einem Polymermaterial.

Als Materialien eignen sich hierbei vor allem Polyurethan, Polyacryl, Polyester, Polytetrafluoroethylen oder Polyethylen, und aufgrund seiner peptidähnlichen Bindungsstruktur vor allem Polyurethan und Polyamid, was eine besonders gute Verankerung ("Anwachsen") des Thrombus an den Fasern ermöglicht.

Neben Polymermaterialien kommen vor allem metallische Materialien in Frage. Geeignete Metalle sind alle Metalle, die ohne Nachteil für die Patienten zur Behandlung herangezogen werden können. Insbesondere geeignet sind für den genannten Zweck Edelstahlfasern aus Metalllegierungen mit Formgedächtniseigenschaften, wie beispielsweise Nitinol-Fasern. Fasern aus Formgedächtnismaterialien haben den Vorteil, daß sie unter dem äußeren Zwang eines Mikrokatheters in einer ersten, eng an das distale Element anliegenden Form vorliegen können und nach Freisetzung aus dem Mikrokatheter in einer zweiten orthogonalen Form mit frei abstehenden Fasern. Weiterhin sind Gold und Platin geeignete Materialien.

Weitere geeignete Materialien sind keramische Materialien und Glasfasern, wobei Kohlenstofffasern zu den keramischen Materialien zu zählen sind.

Die erfindungsgemäß zum Einsatz kommenden Fasern oder Borsten stehen vorzugsweise in einem Winkel von etwa 90° von distalen Element ab. Eine orthogonale Struktur im Sinne der Erfindung ist aber eine jede Struktur, die einen im wesentlichen nicht parallelen Verlauf zum distalen Element hat, d. h. einen beliebigen Winkel zum distalen Element ausbildet.

Es ist darauf zu achten, daß die erfindungsgemäß zum Einsatz kommenden Fasern hinreichend steif sind, um einen Thrombus festzuhalten und an sich zu binden. Die Steifigkeit darf aber nicht so weit reichen, daß die Fasern oder Borsten geeignet sind, die Gefäßwände zu verletzen.

Die Fasern oder Borsten werden auf an und für sich bekannte Art und Weise mit dem distalen Element verbunden, wie dies beispielsweise aus der Fertigung von faserbewehrten Embolisationsspiralen bekannt ist. Dies kann durch Verflechten mit dem distalen Element, durch Verkleben, Verschweißen oder jede andere geeignete Befestigungsart geschehen.

Zur Behandlung besonders kleinlumiger Gefäße eignen sich insbesondere Fasern einer Länge von 0,5 bis 6 und vorzugsweise 0,5 bis 3 mm, so daß auch bei radialer Anordnung der Borsten ein Außendurchmesser des die Fasern tragenden Teils des distalen Elements von 1 bis maximal 12 mm erreicht wird. Zur besonders atraumatischen Behandlung sollte dieser Außendurchmesser etwas kleiner dimensioniert sein als der Innendurchmesser des betreffenden Blutgefäßes.

Gemäß einer zweckmäßigen Ausführungsform sind die Fasern im wesentlichen gerade ausgebildet.

Gemäß einer weiteren zweckmäßigen Ausführungsform sind die Fasern hakenförmig ausgebildet, wobei ihre hakenförmigen Enden zweckmäßigerweise nach proximal gebogen sind, so daß sie beim Verschieben in das Blutgefäß nicht stören und der besonders guten Verankerung des Thrombus beim Herausziehen aus dem Blutgefäß dienen. In Frage kommen weiterhin gekräuselte oder helixartige Strukturen. Soweit die Fasern dazu bestimmt und geeignet sind, mit einem Thrombus zu "verkfeben", ist eine gute Anschmiegbarkeit und große Kontaktfläche von Vorteil.

Gemäß einer vorteilhaften Ausführungsform der Vorrichtung erstrecken sich die Fasern oder Faserbündel radial von der Längsachse des distalen Elements nach außen. Bei dieser Ausführungsform ist der die Fasern tragende Teil des distalen Elements nach Art einer Flaschenbürste ausgebildet.

Gemäß einer weiteren Variante sind die einzelnen Fasern oder Faserbündel in Reihen angeordnet. Dies bedeutet, dass sich die Fasern im wesentlichen parallel zueinander in mehrere Richtungen erstrecken, so dass zwischen diesen Faserreihen und der Faserbündelreihen faserfreie Kanäle frei bleiben.

Gemäß einer weiteren vorteilhaften Ausgestaltung der Vorrichtung sind die Fasern spiralförmig entlang der Längsachse des Filamentes angeordnet. Diese Ausführungsform eignet sich besonders gut zum "Aufspießen" des Thrombus, da der die Fasern tragende Teil des distalen Elements nach Art eines Korkenziehers arbeitet, wenn er vom Operateur entsprechend manövriert wird.

Vorzugsweise beträgt der Winkel zwischen Fasern und distalem Element 45 ° bis 105°, wobei der Wert von 45° mit proximaler Ausrichtung und der von 105° mit distaler Ausrichtung der Fasern gilt.

Besonders bevorzugt beträgt der Winkel zwischen dem distalen Element und der Längsachse der Fasern maximal 90° oder etwas weniger als 90°. Die Ausführungsform mit einem Winkel etwas unter 90° ist dabei besonders atraumatisch beim Vorschieben in das Gefäß bzw. durch den Thrombus und bewirkt gleichzeitig eine besonders gute Verankerung im Thrombus beim Zurückziehen aus dem Blutgefäß.

Gemäß einer zweckmäßigen Ausführungsform der Vorrichtung erstrecken sich die Fasern über eine Länge des distalen Elements, die 0,5 bis 5 cm ausmacht.

Zur Gewährleistung einer ausreichend guten Verankerung des Thrombus, ohne zu große Steifigkeit aufzuweisen, um durch den Thrombus geführt zu werden, ist es zweckmäßig, wenn die Fasern in einer Dichte von 20 bis 100 pro cm an dem distalen Teil der Einführhilfe angeordnet sind.

Grundsätzlich können die Fasern auf jede Art an der Einführhilfe befestigt sein, die deren Ablösung verhindert. Besonders geeignet sind hier das Verkleben oder die mechanische Verbindung. So eignet sich zum Verkleben beispielsweise Permabond. Als mechanische Verbindung kommt vor allem die Verklemmung in der Einführhilfe in Frage, insbesondere, wenn das die Fasern tragende distale Element als Mikrowendel oder -spirale ausgebildet ist.

Zweckmäßigerweise ist der Führungsdraht aus einem medizinischen Edelstahl oder einem Formgedächtnismaterial, vorzugsweise Nitinol ausgebildet.

Es ist weiterhin zweckmäßig, wenn der Führungsdraht einen Außendurchmesser von 0,2 bis 0,4, vorzugsweise 0,22 bis 0,27 mm aufweist.

Darüber hinaus ist es zweckmäßig, wenn der Führungsdraht eine Länge von 50 bis 180 cm aufweist.

Das distale Element kann ebenfalls aus Edelstahl oder einem Formgedächtnismaterial, etwa Nitinol, bestehen. Vorzugsweise besteht das distale Element aber aus einem röntgendichten Material, beispielsweise einer Platinlegierung, oder enthält ein solches Material.

Das distale Element kann langgestreckt mit einem im wesentlichen geraden Verlauf ausgebildet sein, je nach Einsatzzweck aber auch eine gekrümmte Struktur aufweisen, beispielsweise mit einer J-förmig abgebogenen Spitze, einer Korkenzieherstruktur oder dergleichen.

Gemäß einer vorteilhaften Ausführungsform ist das distale Element mit mindestens einem röntgendichten Marker versehen.

Es ist weiterhin vorteilhaft, wenn die Spitze des distalen Elements atraumatisch, also zum Beispiel abgerundet, ausgebildet ist.

Gemäß einer besonders bevorzugten Ausführungsform der Vorrichtung sind die Fasern beschichtet. Dies kann beispielsweise eine neutrale Beschichtung aus Parylen oder Teflon^{®} sein, aber auch eine solche mit Kollagen oder mit einem die Blutgerinnung fördernden Material, vorzugsweise einem oder mehreren Gerinnungsfaktoren. Diese Ausführungsform dient der verstärkten Verankerung der Fasern in dem Thrombus und vermindert die Gefahr, daß der Thrombus zerfällt und Teile des Thrombus im Blutgefäß zurückbleiben oder sich in den Blutstrom freisetzen.

Es wurde überraschend gefunden, daß eine thrombogene Ausrüstung der orthogonalen Struktur und insbesondere der Fasern zu einer erheblichen Stabilisierung an der erfindungsgemäßen Vorrichtung führen. Es ist dabei dem Operateur überlassen, die erfindungsgemäße Vorrichtung so mit dem Thrombus in Kontakt zu bringen und in Kontakt zu belassen, daß über eine gewisse Einwirkungszeit der thrombogenen Elemente ein "Festwachsen" des Thrombus an der Vorrichtung gefördert wird. Dieses "Festwachsen" erfolgt in relativ kurzer Zeit an thrombogenen Fasern/Borsten, teilweise innerhalb einiger Minuten. Dies beugt nicht nur einem Zerfall des Thrombus, wie er mit vielen kommerziell erhältlichen Retrievern beobachtet werden kann, vor, sondern erleichtert auch die Einholung des Thrombus und seine Extraktion aus dem vaskulären System. Die dazu besonders geeigneten thrombogenen Materialien und Beschichtungen sind dem Fachmann aus der Literatur bekannt.

Zu diesem Zwecke eignen sich insbesondere einer oder mehrere der Faktoren Fibrin, Thrombin, Faktor XIII und / oder Faktor VIII.

Darüber hinaus kann es zweckdienlich sein, wenn auch der die orthogonale Struktur tragende Bereich des distalen Elements mit dem oben beschriebenen gerinnungsfördernden Material beschichtet ist.

Die erfindungsgemäße Vorrichtung weist am distalen Element eine längliche Korbstruktur auf, die geeignet ist, sich unter dem äußeren Zwang eines Katheters eng zusammenzufalten und ohne den äußeren Zwang zur vollen Korbstruktur zu entfalten. Die Ausgestaltung derartiger Körbe ist dem zuständigen Fachmann hinlänglich bekannt. Die Korbstruktur kann bei der Extraktion des Thrombus hilfreich sein.

Die Korbstruktur weist im allgemeinen eine längliche, schiffähnliche Struktur mit einer Länge von 5 bis 50 mm und einen Durchmesser von 2 bis 6 mm auf. Der Korb kann halbseitig mit einer Netzstruktur verschlossen sein und/oder periphere, insbesondere längsverlaufende Verstärkungsdrähte oder Streben aufweisen.

Gemäß einer zweckmäßigen Ausführungsform der erfindungsgemäßen Kombination weist auch die Korbstruktur mindestens einen röntgendichten Marker auf. Dieser ist vorteilhafterweise am distalen Ende der Korbstruktur angeordnet.

Weiterhin ist es zweckmäßig, wenn die Korbstruktur aus einem Formgedächtnismaterial, vorzugsweise Nitinol, besteht, so daß sie sich im zusammengefalteten Zustand in einem Mikrokatheter transportieren läßt und nach Herausschieben aus dem Mikrokatheter auffaltet.

Eine besonders bevorzugte Ausführungsform einer solchen Korbstruktur weißt drei oder mehr Streben auf, insbesondere vier Streben, die im Abstand von 90° zueinander angeordnet sind. Diese Streben können durch eine Netzstruktur miteinander verbunden sein, dies ist jedoch nicht unbedingt erforderlich. Die Streben sind vorzugsweise aus einem Formgedächtnismaterial, beispielsweise Nitinol gefertigt.

Die orthogonale Struktur des distale Elements verläuft dabei vorzugsweise zentral in der Korbstruktur. Es sind allerdings Varianten denkbar, die eine asymetrische Verteilung der Streben sinnvoll erscheinen lassen wie auch eine nicht zentrale Anordnung des distalen Elements, beispielsweise dann, wenn es darauf ankommt, einen Thrombus seitlich zu umgehen und in die Korbstruktur hineinzubefördern.

Eine Zusammenfaltung der Korbstruktur unter dem äußeren Zwang eines Katheters tritt im allgemeinen mit einer Streckung der Strukturen im Bereich des distalen Elements auf. Um diese Streckung aufzufangen - bzw. bei der Freisetzung des distalen Elements aus dem Führungskatheter die Kontraktion zu erleichtern, ist das distale Element im Bereich des Korbs beweglich so ausgestattet, dass es dieser Streckung/Kontraktion folgen kann. Dazu weißt das distal Element eine Führung auf, in der sich ein proximales und bevorzugt auch ein distales Drahtelement dieses distalen Elements in axialer Richtung bewegen können. Eine solche Führung besteht zweckmäßigerweise aus einem schraubenförmig gewickelten Draht, der in seinem Inneren einen Hohlraum lässt. Ein solcher Draht kann beispielsweise aus einem radiopaken Metall bestehen, beispielsweise Platin oder einer Platinlegierung.

Bei dieser Ausführungsform ist die orthogonale Struktur in Form von Fasern oder Faserbündeln zweckmäßigerweise an der Führung festgelegt, d. h. mit der Führung verklebt, daran angeschmolzen oder um die Drahtwindungen gewickelt.

Um eine Zwangsaufrichtung der Fasern oder Faserbündel zu ermöglichen, können die Fasern oder Faserbündel an den Streben der Korbstruktur festgelegt sein, beispielsweise durch Verkleben, Anschmelzen, aber auch dadurch, dass die einzelnen Fasern als Schlaufen ausgebildet sind, die um die Streben gelegt werden. Beim Entfalten der Streben nach Freigabe aus einem Katheter kommt es zu einer Zwangsaufrichtung der Fasern beziehungsweise Faserbündel.

Gemäß einer anderen vorteilhaften Ausführungsform verlaufen die Streben der Korbstruktur schraubenlinenförmig, d. h. Anfangspunkt und Endpunkt auf dem distalen Element sind um einen Winkel gegeneinander versetzt, beispielsweise um 45° bis 180°, vorzugsweise um etwa 90°. Ein solcher Schraubenlinienverlauf erlaubt es, beim Vorschieben der Korbstruktur einen an der Gefäßwand haftenden Thrombus abzuschären bzw. abzuschneiden, ohne dass dazu die Vorrichtung gedreht werden müsste.

Gemäß einer anderen Variante verlaufen die Streben in einer Wellenline mit einer seitlichen Auslenkung von 45° bis etwa 90°, d. h. die Streben bewegen sich zunächst seitlich, bis sie beispielsweise einen Punkt erreicht haben, der um 90° gegen den Startpunkt auf dem distalen Element versetzt ist, und bewegen sich anschließend, auf der zweiten Hälfe ihrer Länge zum Ausgangspunkt zurück.

Die Korbstruktur kann, wie oben für die Borsten/Fasern beschrieben, aus den gleichen thrombogenen Materialien bestehen oder damit ausgerüstet sein.

Die Erfindung betrifft schließlich auch die Kombination der Vorrichtung mit einem Führungs- und/oder Mikrokatheter, in dem die Vorrichtung an den Einsatzort manövriert und mit dem Thrombus beladen wieder aus dem Blutgefäßsystem entfernt werden kann. Es kann sinnvoll sein, den Katheter dazu zusätzlich als Aspirationskatheter auszulegen, mit dem Mikrokatheter abgesaugt werden können.

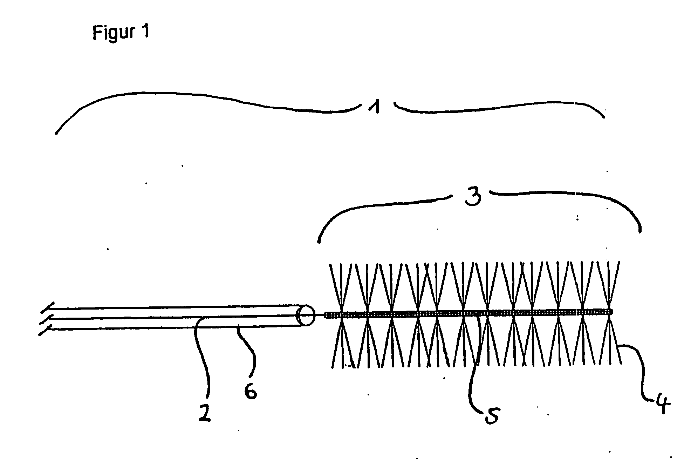

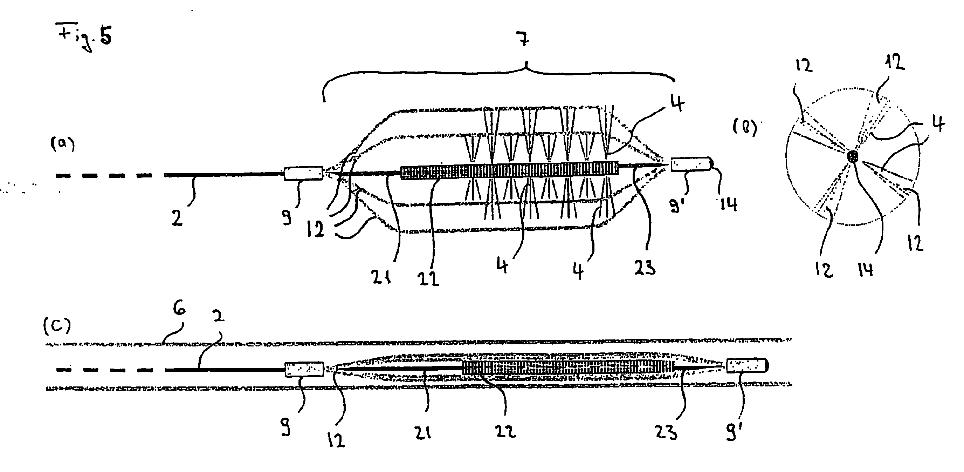

Die Erfindung wird nachfolgend exemplarisch anhand der folgenden Zeichnungen näher erläutert, wobei nur die in den Figs. 5-12 dargestellten Vorrichtungen Ausführungsbeispiele der Erfindung sind, während die in den Figs. 1-4 dargestellten Vorrichtungen nicht in den Schutzbereich der Patentansprüche fallen.

Es stellen dar:
- Figur 1: die vergrößerte Darstellung einer nach Art einer Flaschenbürste ausgestalteten Vorrichtung;
- Figur 2: die Darstellung der Kombination einer Vorrichtung mit einer einen Fangkorb tragenden Vorrichtung in vergrößerter Darstellung;
- Figur 3: vergrößerte Darstellung einer Vorrichtung mit zusätzlichem Fangkorb;
- Figur 4: in vergrößerter Darstellung einen Führungsdraht mit daran angeordnetem Fangkorb;
- Figur 5: eine Ausführungsform einer erfindungsgemäßen Vorrichtung in seitlicher Ansicht, in der Sicht von vorn und in zusammengefalteter Form;
- Figur 6: Verlaufsvarianten für die Streben der Korbstruktur nach Figur 5;
- Figur 7: eine Variante für die Verbindung der orthogonalen Struktur mit den Streben von Figur 5;
- Figur 8: eine weitere Variante der Ausgestaltung eines Fangkorbs der erfindungsgemäßen Vorrichtung;
- Figur 9: einen mit Nylonfasern verspannten Fangkorb gemäß der Erfindung;
- Figur 10: eine weitere Variante der erfindungsgemäßen Vorrichtung mit einem Fangkorb mit Distanzelementen;
- Figur 11: eine weitere Ausführungsform einer erfindungsgemäßen Vorrichtung mit einer eingespannten Polymerhaut; und
- Figur 12: eine weitere Ausführungsform mit über Kreuz verlaufenden Streben des Fangkorbs der erfindungsgemäßen Vorrichtung.

Die in Figur 1 dargestellte Vorrichtung 1 weist am distalen Ende des aus Nitinol gefertigten Führungsdrahtes 2 mit einem Durchmesser von 0,254 mm eine flaschenbürstenartige Funktionseinheit 3 zur Einholung von Thromben auf. Die Funktionseinheit 3 weist Polyamid-Borsten 4 einer Länge von 2 mm auf, die radial an dem Führungsdraht 2 angebracht sind. Der Durchmesser der Funktionseinheit 3 beträgt somit 4 mm und eignet sich insbesondere zur Bergung von Thromben aus Gefäßen von ca. 4,5 bis 5 mm Innendurchmesser. Der Führungsdraht 2 ist in dem die Borsten tragenden Bereich als Platin-Mikrowendel 5 ausgebildet; diese Ausführungsform ist besonders flexibel und dient gleichzeitig als radiopaker Marker für die röntgenologische Kontrolle des Eingriffs. Die distale Spitze der Mikrowendel endet in einem besonders atraumatischen abgerundeten Kopf. Die Borsten 4 sind mechanisch in der Wendel verklemmt, wobei sich jeweils eine zwei Borsten 4 bildende Faser von etwas mehr als doppelter Borstenlänge quer durch die Wendel spannt und so zwei sich gegenüberliegende Borsten ausbildet, die aus der Wendel herausstehen. Die Borsten sind mit Fibrin beschichtet, um eine gute Thrombenadhäsion an der Vorrichtung 1 zu gewährleisten.

Die Vorrichtung 1 wird mit dem distalen Anteil voran mittels eines Mikrokatheters 6 von beispielsweise 0,67 mm Innendurchmesser durch das Blutgefäßsystem in das durch den Thrombus verschlossene Blutgefäß geschoben. Dort wird unter röntgenologischer Kontrolle der korrekten Positionierung durch herkömmliche Verfahren des Standes der Technik die Vorrichtung aus dem Katheter heraus, an dem Thrombus vorbei oder durch diesen hindurch geführt. Die Borsten 4 sind dabei durch den mechanischen Widerstand nach proximal ausgerichtet. Anschließend wird die Vorrichtung 1 nach proximal zurückgezogen, wobei sich die Borsten aufstellen, im Thrombus verhaken und diesen mit sich in den Mikrokatheter 6 führen, worin er aus dem Blutgefäßsystem entfernt wird.

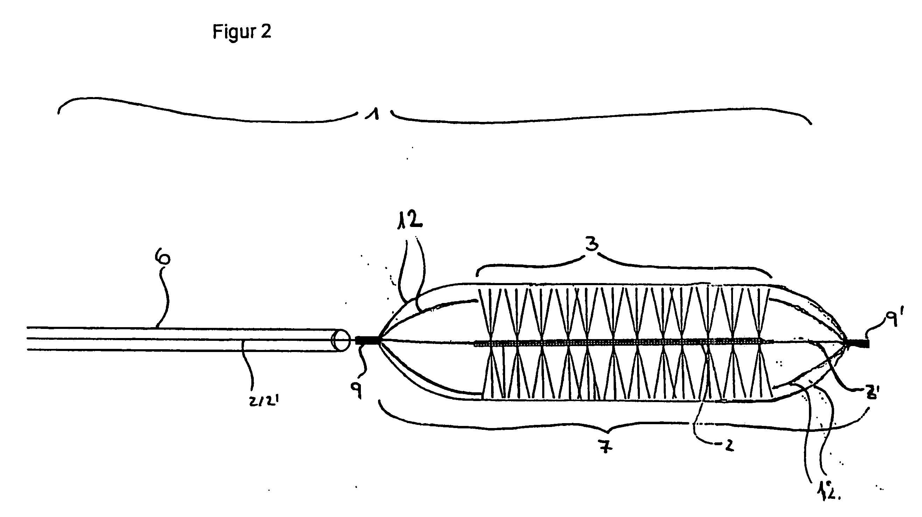

Figur 2 stellt die Kombination einer Vorrichtung 1 mit flaschenbürstenartig ausgestalteter Funktionseinheit 3 mit einer einen aus Streben 12 gebildeten Thromben-Fangkorb 7 tragenden Vorrichtung dar. Beide Vorrichtungen weisen separate Führungsdrähte 2/2' auf, die in der Figur aber nicht separat dargestellt sind. Der Fangkorb 7 ist aus Nitinol ausgebildet und kann (in der Figur nicht dargestellte) Maschen aufweisen, die über seinen Körper verteilt sind. Proximal und distal des Korbes befinden sich radiopake Marker 9 aus Platin an dem Führungsdraht 2' der die Fangkorb 7 tragenden Vorrichtung zur röntgenologischen Detektion. Der Fangkorb 7 wird durch den vom Mikrokatheter 6 ausgeübten mechanischen Zwang während seiner Passage in dem selben im komprimierten Zustand gehalten. Nach Herausschieben aus dem Mikrokatheter 6 durchläuft er infolge des Wegfalls des Zwanges eine spannungsinduzierte martensitische Transformation und nimmt seine expandierte Konfiguration ein, die in der Figur 2 dargestellt ist. In dieser Konfiguration weist er einen Außendurchmesser auf, der zweckmäßigerweise im wesentlichen der der Funktionseinheit 3 der Vorrichtung 1 entspricht oder geringfügig größer, aber kleiner als der Innendurchmesser des zu behandelnden Gefäßes ist. Nachdem der Korb 7 aus dem Mikrokatheter 6 herausgeschoben wurde, wird der distale Bereich der im selben Mikrokatheter 6 geführten Vorrichtung 1 ebenfalls aus dem Mikrokatheter 6 durch den Korb 7 in den Thrombus geführt. Anschließend wird die Vorrichtung 1 mitsamt dem sich in der Funktionseinheit 3 befindenden Thrombus in den Korb 7 gezogen. Sobald sich Funktionseinheit 3 und Thrombus in dem Korb 7 befinden, werden beide Vorrichtungen unter Vermeidung von Relativbewegungen beider Vorrichtungen zu einander gemeinsam in den Mikrokatheter 6 zurückgezogen. Der Korb dient dabei als weitere Sicherung gegen ein Lösen des Thrombus oder von Fragmenten desselben von der Vorrichtung 1.

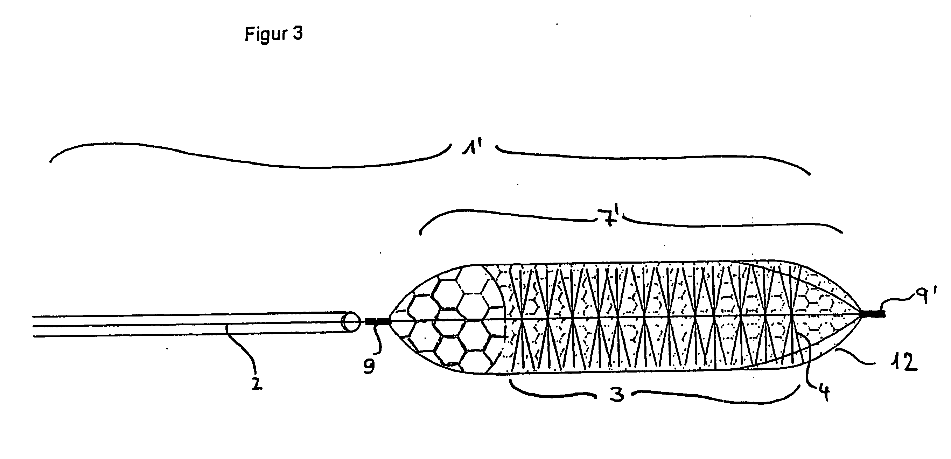

Figur 3 stellt eine weitere Ausführungsform der Vorrichtung 1' mit zusätzlichem Fangkorb 7' dar, der an demselben Führungsdraht angebracht ist, an dem sich auch die flaschenbürstenartig ausgestaltete Funktionseinheit 3 mit den radial angebrachten Borsten 4 befindet. Der Führungsdraht 2 ist hier durchgängig aus Nitinol ausgebildet. Die Borsten 4 sind im Bereich der Funktionseinheit 3 an diesem durch Verkleben mit Permabond befestigt. Die Vorrichtung 1' weist zwei radiopake Marker 9/9' aus Platin auf, die sich proximal und distal des Korbes 7' befinden. Der Fangkorb weist eine Maschenstruktur auf, die sich von proximal zu distal hin verdichtet, so daß eine verbesserte Sicherheit gegen ein Herausrutschen von Thrombus-Fragmenten aus dem Korb beim Zurückführen der Vorrichtung 1' aus dem Blutgefäß gewährleistet ist. Eine größere Maschenweite im proximalen Bereich des Korbs erleichtert das Zurückziehen des Korbs in den Katheter unter Zusammenfallen der Strukturen und Einschluß des Thrombus, der zusätzlich durch die Borsten im Korb fixiert wird.

Es sei darauf hingewiesen, daß die erfindungsgemäße Vorrichtung auch zur Extraktion von Fremdkörpern geeignet ist, beispielsweise von Embolisierungsspiralen oder Stents.

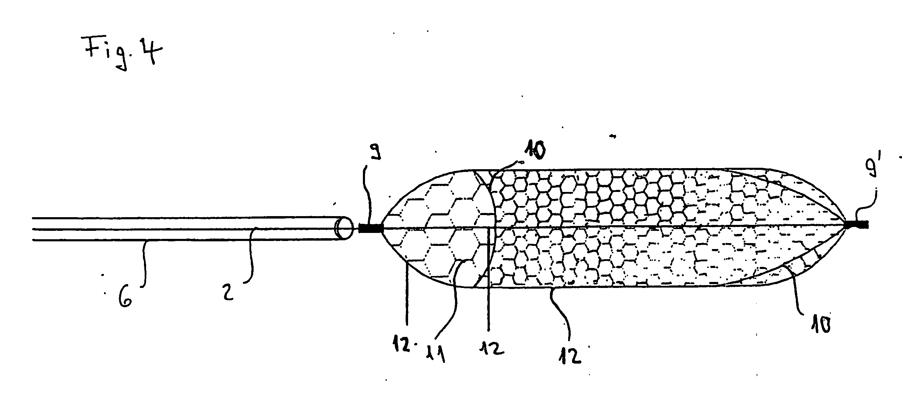

Figur 4 zeigt eine einfache Ausführungsform des Fangkorbs, wie er vorstehend schon in Zusammenhang mit Figur 3 beschrieben ist. Der Fangkorb besteht in diesem Fall aus einer äußeren Drahtkonstruktion und Verstärkungsstreben aus einem Formgedächtnismaterial, etwa Nitinol sowie einem darin angeordneten Netz aus einem thrombogenen Material, beispielsweise Polyurethan, Polyamid oder dergleichen. Die Materialien können auch beschichtet werden. Die Korbstruktur entfaltet sich nach der Freisetzung aus dem Mikrokatheter und ist in der Lage, sich nach Einfangen des Thrombus bei Zurückziehen in den Mikrokatheter wieder zusammenzufalten, begünstigt durch die proximal sich verjüngende Form der Einfassung.

In der Darstellung ist die Drahteinfassung mit 12 bezeichnet, und die Netzstruktur mit 11. Die Verstärkungsstreben sind mit 10 bezeichnet.

Figur 5 zeigt eine Variante einer erfindungsgemäßen Vorrichtung zum Extrahieren von Thromben mit dem Führungsdraht oder Puscherdraht 2, der in die Korbstruktur 7 mit insgesamt vier gleichmäßig über den Umfang verteilten Streben 12 übergeht. Die Streben 12 bestehen aus Nitinol, haben also Formgedächtniseigenschaften. Sie erstrecken sich von einem proximalen Marker 9 zu einem Marker 9', welche gleichzeitig als Hülse ausgebildet sind, die die Streben 12 zur Korbstruktur bündeln. Im gezeigten Fall hat die Korbstruktur vier Streben, die jeweils um 90° Grad gegeneinander versetzt sind; andere Strukturen mit weniger oder mehr Streben und gleichmäßiger oder ungleichmäßiger Verteilung über den Kreis sind ohne weiteres möglich. Der Führungsdraht 2 geht distal zum Marker 9 in einen proximalen Draht 21 über, der in ein schraubenförmig gewickeltes Führungselement 22 hineinragt. Das Führungselement 22 besteht aus einem Platin/Iridium-Draht mit Markereigenschaften. Am distalen Ende dieser Führung 22 befindet sich eine zweites Drahtsegment 23, das im distalen Marker 9' endet. Das distale Drahtsegment 23 kann fest mit der Platinführung 22 verbunden oder aber in ihm geführt sein. Am distalen Ende des distalen Markers 9' befindet sich eine atraumatische Spitze 14 in Form einer Platin- oder Kunststoffhalbkugel.

Das distale Element besteht bei dieser Vorrichtung aus dem proximalen Drahtelement 21, der Führung 22, dem distalen Drahtelement 23 sowie den an der Führung 22 angehefteten Faser- oder Borstenbündeln 4. Die Faserbündel können in die Drahtwicklung 22 eingeflochten oder daran angeklebt sein, gegebenenfalls auch angeschmolzen. Figur 5b zeigt die Anordnung 5a vom distalen Marker 9' hergesehen, mit der atraumatischen Spitze 14 im Zentrum, den im Abstand von 90° angeordneten Streben 12 sowie den um die Streben angeordneten Faserbündeln 4. Die Faserbündel sind im gezeigten Fall in vier Staffeln angeordnet und aneinander ausgerichtet.

Figur 5c zeigt die Vorrichtung von Figur 5a eingezogen in einem Mikrokatheter 6 mit gestreckten Streben 12 und angelegten Fasern 4. Es ist zu erkennen, dass - im Vergleich zur Figur 5a - der Abstand zwischen den Markern 9 und 9' durch die Streckung der Streben 12 größer geworden ist.

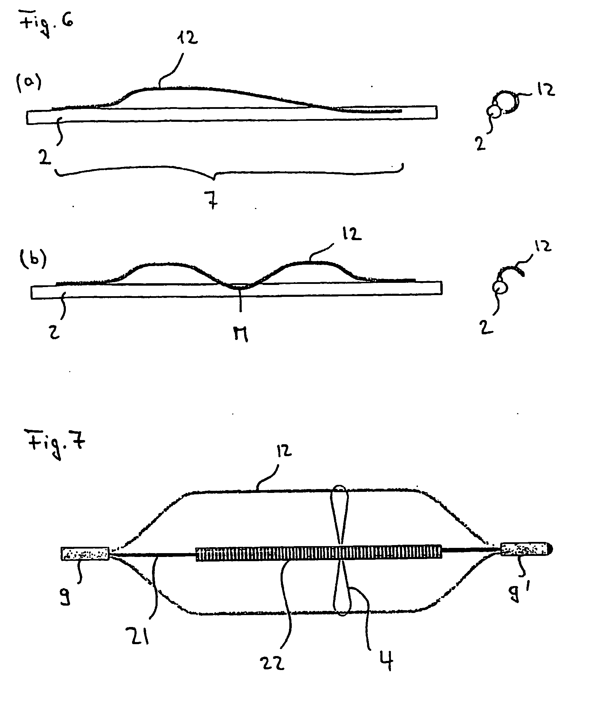

Figur 6 zeigt zwei Varianten einer Strebenführung in Abwandlung der Vorrichtung gemäß Figur 5. In Bezug auf den Führungsdraht 2 beziehungsweise dessen distale Fortsetzung im distalen Element 3 ergibt sich für eine Strebe 12 bei um 90° gegeneinanderersetzten Start- und Endpunkten der gezeigte Drähteverlauf, bei dem die Strebe 12 auf den Betrachter zuläuft. Bei Betrachung

in Richtung des Führungsdrahtes 2 ergibt sich für den Verlauf der Strebe 12 in etwa ein Dreiviertelkreis wobei zu berücksichtigen ist, dass Startpunkt und Endpunkt durch die Länge 7 der Korbstruktur voneinander getrennt sind.

Ein derartiger Verlauf der Strebe 12 ermöglicht dem behandelnden Arzt die Abschärung eines an der Gefäßwand anhaftenden Thrombus durch vorsichtiges Vorschieben der Fangvorrichtung.

Figur 6b zeigt ein weitere Variante, bei der die Strebe 12 zunächst mit einer 90° Drehung auf den Betrachter zuläuft, dort ihren Führungsdraht fernsten Punkt in etwa der Mitte bei M erreicht und anschließend wieder zurückläuft, so dass Start- und Endpunkt keine Versetzung gegeneinander zeigen. Diese Variante hilft dabei, Drehbewegungen und Thrombenbelastungen bei Zug oder Schub zu vermeiden und bietet gleichzeitig einen Längenpuffer.

Figur 7 zeigt schematisch, eine Variante zur Verbindung der Fasern 4 mit den Streben 12 der Korbstruktur. Die Fasern 4 gehen von den Windungen der Führungen 22 aus, schwingen sich um eine Strebe 12 und laufen wieder zur Führung 22 zurück. Bei der Entfaltung der Korbstruktur nach Ausbringung aus einem Katheter erfolgt hierdurch eine Zwangsaufrichtung der Fasern 4 orthogonal zum Verlauf der Führung 22.

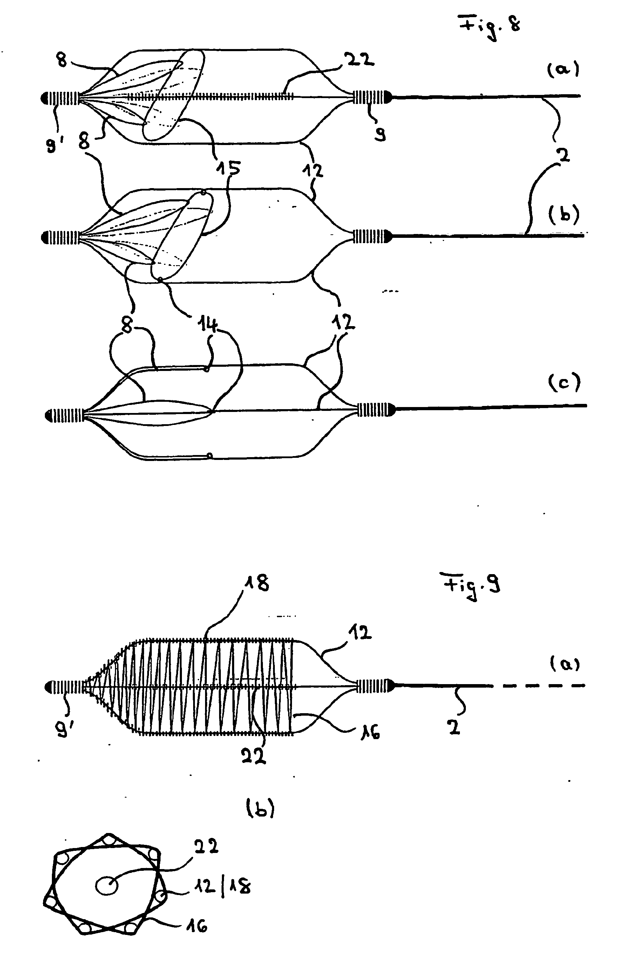

Die Figuren 8 zeigen drei verschiedene Varianten einer Ausführungsform, in der der Korb 7 an seinem distalen Ende zusätzliche Streben oder Schlaufen 8 aufweisen, die das distale Ende des Korbes dichter gestalten. Aus Gründen der Übersichtlichkeit sind bei den Körben nur jeweils zwei von mindestens vier Streben 12 dargestellt, um das Prinzip zu verdeutlichen. Des weiteren sind die zentralen Elemente der "Flaschenbürste" ganz oder teilweise fortgelassen.

Figur 8a zeigt den Korb mit den Streben 12 und seinen Markierungen 9' (distal) und 9 (proximal). Der Führungsdraht 2 verläuft durch die proximale Markierung 9 und geht in die Führung 22 über. An den Streben 12 festgelegt ist ein ovaler Ring 15, um den schlaufenförmig ausgebildete Streben 8 geführt sind. Diese schlaufenförmigen Streben 8 beginnen und enden innerhalb der Markierungsspirale 9', die gleichzeitig als Manschette für die Streben 12 und 8 dient und sind lose um den Ring 15 herum geführt. Die ovale Ausbildung des Rings 15, bei der die Festlegungspunkte an den Streben 12 nicht auf gleicher Höhe liegen, erlaubt eine einfache Anpassung der Streben oder Schlaufen 8 an den Streckungszustand des Korbes innerhalb und außerhalb eines Katheters.

Es versteht sich, dass in dieser und in anderen Ausführungsformen die Markierungsspirale 9 und 9' jeweils eine duale Funktion haben. Zum einen dienen sie als Marker, zum anderen als Manschetten zur Verbindung der Streben 8 und 12, die innerhalb und ggf. auch mit dieser Manschette form- oder stoffschlüssig verbunden sind.

Figur 8b zeigt eine weitere Ausführungsform, in der der ovale Ring 15 durch Ösen 14 an den Streben 12 gezogen ist. Im übrigen entspricht diese Ausführungsform der von Figur 8a.

Figur 8c verzichtet auf den Ring 15 und verbindet die Schlaufen 10 unmittelbar mit Ösen 14 der Streben 12. Es versteht sich, dass die Schlaufen oder Streben 10 sich in ihrem Verlauf an die Korbstruktur anpassen, d.h. einen Verlauf aufweisen, der dem der Streben 12 entspricht.

Weitere Ausführungsformen sind in Figur 9 dargestellt, die aus Gründen der Einfachheit ebenfalls nur einen Teil der Streben darstellen. Die orthogonale Struktur ist in Figur 9a und Figur 9b nicht dargestellt.

Figur 9a zeigt eine erfindungsgemäße Korbstruktur, in der der Führungsdraht 2 durch den proximalen Marker 9 hindurch in die Führung 22 übergeht und im distalen Marker 9' endet. Die Streben 12 weisen über einen Teil ihrer Länge, insbesondere im distalen Bereich eine Wicklung oder Ummantelung auf, die aus Markierungsspiralen aus Platin-Iridium-Legierung besteht, sowie eine Umwicklung mit Fasermaterial 16, die vom distalen Ende her einen Teil der Länge der Streben 12 erfasst. Diese Wicklung kann beispielsweise aus Nylonfasern bestehen, die in die Windungslücken der Markierungsspirale gepresst werden und wird vorzugsweise in einem Zug vorgenommen, d.h. die gesamte Wicklung besteht aus einem Filament. Das Einpressen oder auch Ankleben an die Markierungsspiralen 18 auf den Streben 12 bewirkt gleichzeitig eine Fixierung der Streben in Ihrer Position und ihrem Abstand relativ zueinander.

Figur 9b zeigt eine weitere Variante, in der ein Korb mit insgesamt sieben Streben 12 mit einem Nylonfaden umwickelt ist. Die Umwicklung, die wie in Figur 9a nur einen Teil des Korbs umfasst, nämlich den distalen Bereich, ist hier alternierend innen und außen um die Streben 12 gelegt und bewirkt eine noch weitergehende Fixierung. Die Bezugsziffer 22 bezeichnet die zentrale Führung, die die einzelnen Elemente des Führungsdrahts 2 umgibt. Die Streben 12 weisen dabei eine Markierungsspirale 18 auf.

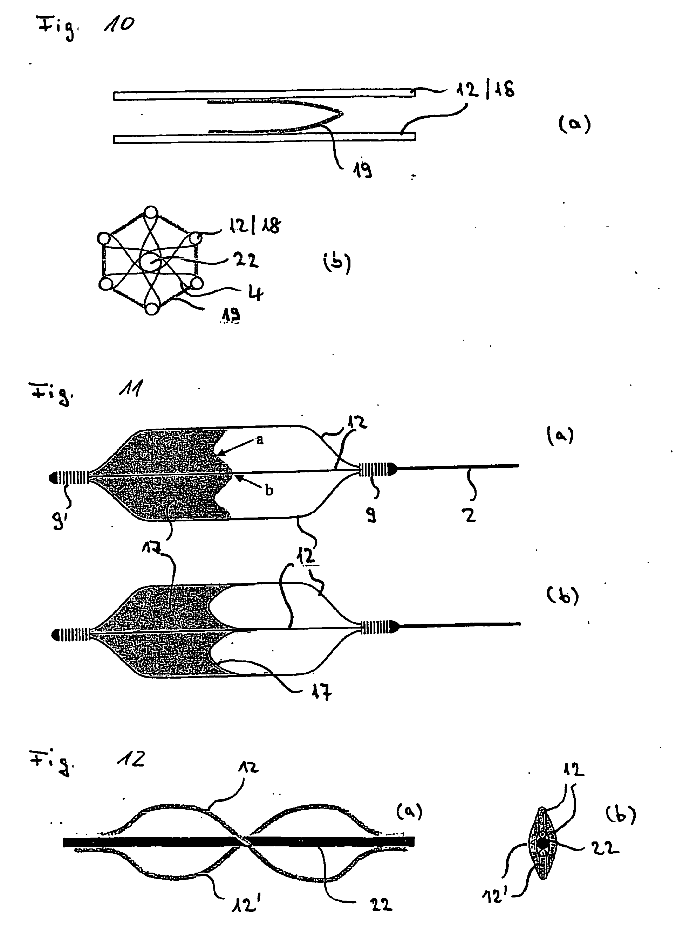

Eine weitergehende Fixierung der Streben 12 in ihrer Position zueinander ist in Figur 10a dargestellt, in der zwei Streben 12 gezeigt werden, die über eine flexible Spange 19 miteinander verbunden sind. Die Verbindung mit diesem "V-Stück" kann entweder form- oder auch stoffschlüssig (Schweißen, Kleben) erfolgen. Die Streben 12 sind auch hier mit Markierungsspiralen aus Platin-Iridium-Legierung ummantelt, welche dazu dienen können, die Spange 19 zu fixieren.

In Figur 10b ist die Ausführungsform von Figur 10a im Querschnitt anhand eines kompletten Korbes dargestellt. Die einzelnen Streben 12, in diesem Falle sechs, sind über die Spangen 19 miteinander verbunden. Zwischen der zentralen Führung 22, die ihrerseits aus einer Spirale besteht, und den Streben 12 sind die Filamente 4 der orthogonalen Struktur geführt, die in Schlaufen um die jeweiligen Längselemente 12 und 22 gelegt sind.

Figur 11 zeigt eine weitere Variante, die an die Varianten mit den Netzstrukturen von Figur 3 und 4 anknüpft. In diesem Fall ist der Korb in seinem distalen Bereich mit einer Polymerhaut 17 aus beispielsweise expandiertem PTFE bespannt, die das distale Ende des Korbs zu einer Tasche werden lässt. Die Polymerhaut erstreckt sich von der distalen Spitze des Korbs entlang der Streben bis zu einer gewünschten Position, beispielsweise etwa in der Mitte des Korbs.

Die proximale Kante der Polymerhaut verläuft dabei vorzugsweise wellenförmig, so dass der Kantenbereich zwischen zwei Streben näher distal liegt (a) als der Bereich an den Streben selbst (b). Die Streben können in die Polymerhaut eingebettet oder an diese angeklebt sein.

Figur 11b zeigt eine weitere Variante mit einem gegenüber Figur 11a geänderten Verlauf der Polymerhautkante, die an den Streben 12 mehr nach distal gezogen ist. Hierdurch wird eine weitgehende Fixierung der Streben zueinander erhalten. Zwischen den Streben ist die Polymerhaut nach proximal eingetieft. Eine Stabilisierung dieser Kantenstruktur kann durch eine Verbindungsstrebe oder Spange 17 erfolgen, wie sie in Figur 10a beschrieben ist.

Figur 12 zeigt schließlich eine besondere Form des Verlaufs der Streben 12, die von distal nach proximal um 180° gegeneinander versetzt verlaufen, d.h. von der Oberseite einer Korbstruktur zur Unterseite. Die Anordnung eines Strebenpaars in Relation zur Führung 22 ist in Figur 12b dargestellt mit einem Strebenpaar 12 und 12'. Es versteht sich, dass die Korbstruktur, die hier eigentlich eine Doppelkorbstruktur ist, aus wenigstens zwei, besser wenigstens drei Strebenpaaren besteht, die regelmäßig über den Umfang verteilt sind.

Die Konstruktion erlaubt es, radiale Kräfte in den Streben durch die Führung auf die gegenüberliegende Seite so zu nutzen, dass der Korb in jedem Fall seine aufgespannte Form erhält, auch dann, wenn der Nenndurchmesser in einem engen Gefäß nicht realisiert werden kann. Dies gilt insbesondere in Verbindung mit der Führung 22 und der dadurch ermöglichten Längenanpassung.

Es versteht sich, dass in der vorliegenden Ausführung die Begriffe "distal" und "proximal" auf den behandelnden Arzt bezogen sind. Damit bezeichnet "distat" das dem behandelnden Arzt abgewandte Ende beispielsweise des Korbs oder des Katheters.

## Patentansprüche

1. Vorrichtung zur Entfernung von Fremdkörpern und Thromben aus Körperhohlräumen und Blutgefäßen mit einem Führungsdraht (2), der ein distales Element (3) aufweist, das mit einer orthogonalen Struktur (4) ausgestattet ist, wobei die Vorrichtung eine längliche Korbstruktur (7) aufweist, die geeignet ist, sich unter dem äußeren Zwang eines Mikrokatheters (6) eng zusammenzufalten, um innerhalb des Mikrokatheters (6) transportiert zu werden und sich bei Wegfall des äußeren Zwangs durch den Mikrokatheter (6) zur vollen Korbstruktur entfaltet,
**dadurch gekennzeichnet**, dass das distale Element (3) des Führungsdrahts (2) im Bereich des Korbs (7) eine Führung (22) aufweist, in der ein proximales Drahtelement (21) des distalen Elements (3) verschiebbar gelagert ist, so dass das distale Element (3) gestreckt und kontrahiert werden kann.

2. Vorrichtung nach Anspruch 1, **dadurch gekennzeichnet, dass** die Korbstruktur (7) drei oder mehr Streben (12) aufweist.

3. Vorrichtung nach Anspruch 2, **dadurch gekennzeichnet, dass** die Streben (12) aus Formgedächtnismaterial bestehen.

4. Vorrichtung nach Anspruch 3, **dadurch gekennzeichnet, dass** die Streben (12) aus Nitinol bestehen.

5. Vorrichtung nach Anspruch 1, **dadurch gekennzeichnet, dass** die Führung (22) ein schraubenförmig gewickelter Draht ist.

6. Vorrichtung nach Anspruch 5, **dadurch gekennzeichnet, dass** der schraubenförmig gewickelte Draht aus Platin oder einer Platinlegierung besteht.

7. Vorrichtung nach einem der Ansprüche 2 bis 4, **dadurch gekennzeichnet, dass** die Streben schraubenlinienförmig verlaufen, so dass ihr Startpunkt gegenüber ihrem Endpunkt um 45° bis 180° versetzt ist.

8. Vorrichtung nach einem der Ansprüche 2 bis 4, **dadurch gekennzeichnet, dass** die Streben entlang einer Wellenlinie mit einer seitlichen Auslenkung von 45° bis 90° verlaufen, wobei Startpunkt und Endpunkt nicht gegeneinander versetzt sind.

9. Vorrichtung nach einem der Ansprüche 2 bis 4, **dadurch gekennzeichnet, dass** die Streben (12) in ihrem Verlauf um 180° versetzt sind.

10. Vorrichtung nach einem der Ansprüche 1 bis 8, **dadurch gekennzeichnet, dass** die Korbstruktur (7) eine Netzstruktur aufweist.

11. Vorrichtung nach einem der Ansprüche 1 bis 8, **dadurch gekennzeichnet, dass** die Korbstruktur (7) distal mit einer Polymerhaut (17) bespannt ist.

12. Vorrichtung nach einem der vorstehenden Ansprüche, in Kombination mit einem Führungskatheter und/oder Mikrokatheter (6).

13. Kombination nach Anspruch 12, **dadurch gekennzeichnet, dass** der Führungs-/Mikrokatheter (6) als Aspirationskatheter ausgelegt ist.

## Claims

1. Device for the removal of foreign bodies and thrombi from body cavities and blood vessels using a guide wire (2) provided with a distal element (3), said distal element (3) having an orthogonal structure (4), wherein said device has an oblong cage structure (7) suited to be flatly collapsible under the external strain exerted by a micro-catheter (6) and capable of being transported inside the micro-catheter (6) and unfolding to its full cage structure when said external strain caused by the micro-catheter (6) is omitted,
**characterized in that** the distal element (3) of the guide wire (2) is provided with a guide (22) in the area of the cage (7) in which a proximal wire element (21) of the distal element (3) is slidably arranged, so as to extend and contract the distal element (3).

2. Device according to claim 1, **characterized in that** the cage structure (7) has been provided with three or more braces (12).

3. Device according to claim 2, **characterized in that** the braces (12) are made of a shape-memory material.

4. Device according to claim 3, **characterized in that** the braces (12) consist of nitinol.

5. Device according to claim 1, **characterized in that** the guide (22) is a wire of helically wound shape.

6. Device according to claim 5, **characterized in that** the helically wound wire consists of platinum or a platinum alloy.

7. Device according to any one of the claims 2 to 4, **characterized in that** the braces extend in the form of a helical line so that their starting point is offset by 45 ° to 180° in relation to their end point.

8. Device according to any one of the claims 2 to 4, **characterized in that** the braces extend in the form of a wave line with a lateral deflection of between 45° and 90° with the starting point and end point not being offset against each other.

9. Device according to any one of the claims 2 to 4, **characterized in that** the braces (12) extend in a configuration offset by 180°.

10. Device according to any one of the claims 2 to 4, **characterized in that** the cage structure (7) has been provided with a net structure.

11. Device according to any one of the claims 1 to 8, **characterized in that** the cage structure (7) has been covered distally with a polymer structure (17).

12. Device according to any one of the above claims, in combination with a guiding catheter and/or micro-catheter (6).

13. Combination according to claim 12, **characterized in that** the guiding/micro-catheter (6) is designed as a aspiration catheter.

## Revendications

1. Dispositif pour éliminer des corps étrangers et des thrombus à partir de cavités corporelles et de vaisseaux sanguins, comportant un fil-guide (2), qui comprend un élément distal (3), lequel est muni d'une structure orthogonale (4), le dispositif présentant une structure en panier oblong (7), qui est conçue pour se réduire par repliement sous l'effet d'une force extérieure exercée par un micro-cathéter (6), pour pouvoir être transporté à l'intérieur du micro-cathéter (6), et qui, quand la force extérieure du micro-cathéter (6) n'est plus appliquée, se déploie jusqu'à une structure de panier complète, **caractérisé en ce que** l'élément distal (3) du fil-guide (2) comprend dans la zone du panier (7) un élément de guidage (22), dans lequel un élément de fil proximal (21) de l'élément distal (3) est logé de façon à pouvoir subir une translation, de telle sorte que l'élément distal (3) puisse subir un déploiement et une contraction.

2. Dispositif selon la revendication 1, **caractérisé en ce que** la structure de panier (7) comprend trois entretoises (12), ou plus.

3. Dispositif selon la revendication 2, **caractérisé en ce que** les entretoises (12) sont constituées d'un matériau à mémoire de forme.

4. Dispositif selon la revendication 3, **caractérisé en ce que** les entretoises (12) sont constituées de nitinol.

5. Dispositif selon la revendication 1, **caractérisé en ce que** l'élément de guidage (22) est un fil enroulé en hélice.

6. Dispositif selon la revendication 5, **caractérisé en ce que** le fil enroulé en hélice est constitué de platine ou d'un alliage de platine.

7. Dispositif selon l'une des revendications 2 à 4, **caractérisé en ce que** les entretoises ont une forme hélicoïdale, de telle sorte que leur point de départ soit décalé de 45 à 180° par rapport à leur point final.

8. Dispositif selon l'une des revendications 2 à 4, **caractérisé en ce que** les entretoises courent le long d'une ligne ondulée ayant un déplacement latéral de 45 à 90°, le point de départ et le point final n'étant pas décalés l'un par rapport à l'autre.

9. Dispositif selon l'une des revendications 2 à 4, **caractérisé en ce que** les entretoises (12) présentent entre elles un décalage de 180°.

10. Dispositif selon l'une des revendications 1 à 8, **caractérisé en ce que** la structure de panier (7) présente une structure réticulée.

11. Dispositif selon l'une des revendications 1 à 8, **caractérisé en ce que** la structure de panier (7) est tendue en position distale à l'aide d'une peau polymère (17).

12. Dispositif selon l'une des revendications précédentes, en combinaison avec un cathéter de guidage et/ou un micro-cathéter (6).

13. Combinaison selon la revendication 12, **caractérisée en ce que** le cathéter de guidage/le micro-cathéter (6) est conçu comme un cathéter d'aspiration.
